# EUROPEAN PATENT APPLICATION

(11) **EP 3 438 664 A1**
(43) Date of publication of application: **06.02.2019**
(21) Application number: 17775358.9
(22) Date of filing: 30.03.2017
(51) Int. Cl.: G01N 33/574, G01N 33/543

(54) **ONCOFETAL FIBRONECTIN DETECTION METHOD USING SIMPLE IMMUNOASSAY**

(30) Priority: 31.03.2016 JP 2016070705
(71) Applicant: Sekisui Medical Co., Ltd., Tokyo 103-0027 (JP)
(72) Inventor: ITOU, Kanako, Tokyo 103-0027 (JP); MORITA, Motoki, Tokyo 103-0027 (JP); YAJI, Shouhei, Tokyo 103-0027 (JP); SAKAI, Shun, Tokyo 103-0027 (JP)
(74) Representative: Hoffmann Eitle
(86) International application number: PCT/JP2017/013151
(87) International publication number: WO 2017/170824

(57) **Abstract**

The present invention addresses the problem of making it possible, in a simple immunoassay, to prevent false-positive reactions and reduce measurement value errors so as to accurately detect fFN by suppressing a cross-reaction with plasma FN that is an antigenic component similar to fFN to reduce the detection of plasma FN. When an anti-fFN antibody reacts with fFN in the presence of a specific surfactant according to the present invention, it is possible to accurately detect fFN through the suppression of the cross-reaction between the antibody and FN, even when other FNs such as plasma FN are also present. The specific surfactant is represented by general formula (1):

R-O-(CH₂CH₂O)ₘH (1)

In the formula, m is 4-30, and R represents an alkyl group.

## Description

### TECHNICAL FIELD

The present disclosure relates to a detection method of oncofetal fibronectin using simple immunoassay, and particularly, to a detection method of oncofetal fibronectin using immunochromatography. Further, the present invention relates to a method capable of accurately detecting oncofetal fibronectin in the case where fibronectins other than oncofetal fibronectin may coexist in a sample.

### BACKGROUND ART

Point of Care Testing (POCT) is a collective term for tests generally conducted near a patient, such as a general practitioner, a hospital ward, or a clinic for outpatient treatment, etc. Since diagnosis and treatment may be quickly performed according to test results obtained by a simple manipulation, it is expected to contribute to the quality improvement of medical treatment.

A detection method using immunochromatography which is a method frequently used in POCT is practically used in a variety of fields, including infectious diseases such as influenza infection, obstetrics such as pregnancy, preterm birth, etc., lifestyle diseases such as diabetes, heart diseases, etc.

A heterogeneous method includes a step of washing components in sample other than an antigen-antibody complex derived from an analyte prior to detection of the analyte during the operation (hereinafter, also simply referred to as a heterogeneous method). On the other hand, a homogeneous method does not include the corresponding washing step (hereinafter, also simply referred to as homogeneous method) . The detection method using immunochromatography corresponds to a homogeneous method.

In the case where a substance having antigenicity similar to an analyte (hereinafter, also referred to as a similar antigen) exists in a sample (specimen), the substance may cause an error to a measurement result in the homogeneous method, even if it exists in an amount that does not cause problems in the heterogeneous method.

An analyte possibly containing similar antigens due to contamination of a sample with blood or the like may be exemplified by fibronectin. Fibronectins constitute a family of proteins expressed from a single gene. It is known that various isoforms of fibronectin are present in plasma and living tissues, including connective tissue, skin, colon, liver, spleen, and kidney (Matsuura and Hakomori, Proc. Natl. Acad. Sci. USA 82: 6517-6521 (1985)). Fetal tissues and several different kinds of tumor cells include fibronectin isoforms collectively called "fetal" or "oncofetal" fibronectins. Oncofetal fibronectin (hereinafter, also simply referred to as "fFN") is rarely present in vaginal secretions of non-pregnant women or pregnant women after 22 weeks of gestation who have no disorders in the fetal membrane, but oncofetal fibronectin leaks into the vaginal secretions when the fetal membrane is damaged or weakened by bacterial infections or physical factors. Therefore, fFN concentrations in the vaginal secretions of pregnant women at 22 weeks to 33 weeks of gestation are useful as a marker for threatened premature labor. Such an oncofetal fibronectin tested in obstetrics is initially written as "oncofetal fibronectin", and in Japanese, it has been translated and used as "Gantaijisei" fibronectin. Currently, an expression of "fetal" fibronectin tends to be used. Unless otherwise specified in the present invention, the terms are unified by the expression of "oncofetal fibronectin".

As described above, fibronectin is a family of proteins expressed from a single gene and is a major protein with a molecular weight of about 500 kDa which constitutes the extracellular matrix. fFN has the same amino acid sequence as plasma-derived FN (hereinafter, also abbreviated to plasma FN or FN), and they are different from each other only in the sugar chain binding site. fFN has a sugar content of about 9.5% and plasma FN has a sugar content of 5.8%. As described above, since fFN and plasma FN have the same amino acid sequence and there is only one fFN-specific epitope currently identified, it is very difficult to obtain two or more kinds of antibodies that specifically react with fFN, but completely do not react with plasma FN.

Therefore, as two antibodies to form a sandwich complex, a pair of antibodies may be needed, one antibody that specifically binds to fFN but does not bind to the other fibronectin and the other antibody that binds to both fibronectins.

Practically, in order to obtain a monoclonal antibody that specifically binds to fFN, the applicant attempted to obtain the monoclonal antibody by using a peptide containing a sugar chain moiety peculiar to fFN as an immunogen, and as a result, the obtained monoclonal antibody was an antibody that specifically reacts with fFN, but shows a slight cross-reactivity with plasma FN.

As a reagent that is currently being distributed as an *in vitro* diagnostic medical product of fFN, "immunotester (registered trademark) fFN" (manufactured by Sekisui Medical Co., Ltd.) which is a reagent employing an ELISA method is known (Non-Patent Document 1).

The immunotester employs a two-step sandwich ELISA method using an immunoplate and employs a heterogeneous method including a washing step. This reagent has a cut-off value of 50 ng/mL, based on the risk of threatened premature labor. Even though a specimen is contaminated with blood, false positive reactions hardly occur and no clinical problems are caused, because of the washing step.

Meanwhile, since a detection method using immunochromatography is a homogeneous method including no washing step, there is a problem that this method is vulnerable to blood contamination, etc. For example, a specimen with blood contamination of 0.1% or more may be distinguished by the color, but it is difficult to visually distinguish a specimen with blood contamination of less than 0.1%. Therefore, a reagent which does not cause a false positive reaction even when blood contamination is less than 0.1% is desirable.

As a method of detecting human fFN using immunochromatography, those in Patent Documents 1 and 2 are known. However, there are no commercialized reagents that specifically detect fFN. Patent Document 1 is a document that discloses general immunochromatography, and this document merely mentions fFN as an analyte. Patent Document 2 discloses addition of BSA, etc. to a sample in order to inhibit non-specific binding of anti-fFN antibody with a background material.

Further, there is no description or suggestion of problems associated with antigen similarity between fFN and other fibronectins in any of Patent Documents 1 and 2.

### CITATION LIST

### PATENT LITERATURE

Patent Document 1: Japanese Patent Application Laid-Open No. 2008-514900
Patent Document 2: Japanese Patent Application Laid-Open No. 2011-39068

### NON-PATENT LITERATURE

Non-Patent Document 1: Attached document for in vitro diagnostic medical product, "immunotester (registered trademark) fFN" (Sekisui Medical Co., Ltd.)

### SUMMARY OF INVENTION

### TECHNICAL PROBLEM

When fFN in a sample possibly contaminated with blood is detected using immunochromatography, plasma-derived fibronectin is also detected, which may cause a problem of obtaining measurement value errors or false positive results.

In order to solve the above problem, an object of the present invention is to provide a detection method using immunochromatography, which suppresses cross-reactivity with other FNs which are antigenic components similar to fFN, reduces detection of similar antigens, and prevents measurement value errors and false positive reactions, thereby allowing accurate detection of fFN.

### SOLUTION TO PROBLEM

As a result of intensive studies to solve the above problems, it was found that when fFN and anti-fFN antibody are allowed to react in the presence of a specific surfactant, cross-reactivity between the antibody and FN may be suppressed and fFN may be accurately detected even though other FNs such as plasma FN coexist, thereby completing the present invention.

Specifically, the present invention is as follows:
(1) A detection method of oncofetal fibronectin using immunochromatography, comprising steps (A) to (C):
   (A) supplying a sample possibly containing oncofetal fibronectin and fibronectins other than oncofetal fibronectin and a liquid composition containing a non-ionic surfactant represented by Formula (1) to a sample-supplying portion of the following test strip;
      the test strip comprising a membrane consisting of a porous body equipped with at least the sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, and wherein any one of the first antibody and the second antibody is an anti-oncofetal fibronectin antibody and the other is an antibody binding to oncofetal fibronectin;
   (B) bringing the sample into contact with the conjugate; and
   (C) detecting a complex of oncofetal fibronectin in the sample and the conjugate in the detecting portion:

      R-O-(CH₂CH₂O)ₘH (1)

      wherein m is 4 to 30, and R represents an alkyl group.
(2) The detection method of (1), wherein a concentration of the surfactant in the liquid composition is in the range of more than 0.2%(W/V) and less than 1.0%(W/V).
(3) A method of reducing cross-reaction between oncofetal fibronectin and fibronectins other than oncofetal fibronectin, in a method of detecting oncofetal fibronectin in a sample using an immunochromatographic test strip,
   the method of reducing cross-reaction comprising the following steps:
   (A) supplying a sample possibly containing oncofetal fibronectin and fibronectins other than oncofetal fibronectin and a liquid composition containing a non-ionic surfactant represented by Formula (1) to a sample-supplying portion of the following test strip;
      the test strip comprising a membrane consisting of a porous body equipped with at least the sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, and wherein any one of the first antibody and the second antibody is an anti-oncofetal fibronectin antibody and the other is an antibody binding to oncofetal fibronectin;
   (B) bringing the sample into contact with the conjugate; and
   (C) detecting a complex of oncofetal fibronectin in the sample and the conjugate in the detecting portion:

      R-O-(CH₂CH₂O)ₘH (1)

      wherein m is 4 to 30, and R represents an alkyl group.
(4) The method of reducing cross-reactivity of (3), wherein a concentration of the surfactant in the liquid composition is in the range of more than 0.2%(W/V) and less than 1.0%(W/V).
(5) An immunochromatographic detection kit for oncofetal fibronectin, comprising the following components (i) and (ii) :
   (i) an immunochromatographic test strip comprising a membrane consisting of a porous body equipped with at least a sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, and wherein any one of the first antibody and the second antibody is an anti-oncofetal fibronectin antibody and the other is an antibody binding to oncofetal fibronectin; and
   (ii) a liquid composition containing a nonionic surfactant represented by the following Formula (1):

      R-O-(CH₂CH₂O)ₘH (1)

      wherein m is 4 to 30, and R represents an alkyl group.
(6) The detection kit of (5), wherein the liquid composition of (ii) is used as a spreading solution or a specimen extraction solution.
(7) The detection kit of (5) or (6), wherein a concentration of the surfactant in the liquid composition is in the range of more than 0.2%(W/V) and less than 1.0%(W/V).
(8) A liquid composition for detecting oncofetal fibronectin using immunochromatography, comprising a nonionic surfactant represented by the following Formula (1):

   R-O-(CH₂CH₂O)ₘH (1)

   wherein m is 4 to 30, and R represents an alkyl group.
(9) The liquid composition of (8), wherein the liquid composition is used as a spreading solution or a specimen extraction solution.
(10) The liquid composition of (8) or (9), wherein a concentration of the surfactant in the liquid composition is in the range of more than 0.2%(W/V) and less than 1.0%(W/V).
(11) A specimen extraction solution for detecting oncofetal fibronectin, comprising a nonionic surfactant represented by the following Formula (1):

   R-O-(CH₂CH₂O)ₘH (1)

   wherein m is 4 to 30, and R represents an alkyl group.
(12) A method of detecting oncofetal fibronectin using immunochromatography, comprising bringing a sample possibly containing oncofetal fibronectin and fibronectins other than oncofetal fibronectin into contact with an anti-oncofetal fibronectin antibody in the presence of a nonionic surfactant represented by the following Formula (1):

   R-O-(CH₂CH₂O)ₘH (1)

   wherein m is 4 to 30, and R represents an alkyl group.

### ADVANTAGEOUS EFFECTS OF INVENTION

In a method of detecting fFN (oncofetal fibronectin) in a sample using immunochromatography, even when the sample is contaminated with blood and thus plasma FN which is an antigen similar to fFN coexists, cross-reactivity between an anti-fFN antibody and plasma FN may be reduced, and measurement errors or false positive reactions may be prevented, thereby allowing accurate detection of fFN.

### BRIEF DESCRIPTION OF DRAWINGS

Fig. 1 is a graph showing experimental results of examining effects of a variety of different nonionic surfactants on cross-reactivity between an anti-fFN antibody and plasma FN;
Fig. 2 is a graph showing experimental results of examining effects of a difference in an addition concentration of Brij on cross-reactivity between an anti-fFN antibody and plasma FN;
Fig. 3 is a graph showing experimental results of examining effects of a difference in chain length of Brij on cross-reactivity between an anti-fFN antibody and plasma FN;
Fig. 4 is a graph showing experimental results of examining effects of a difference in chain length of EMULGEN on cross-reactivity between an anti-fFN antibody and plasma FN; and

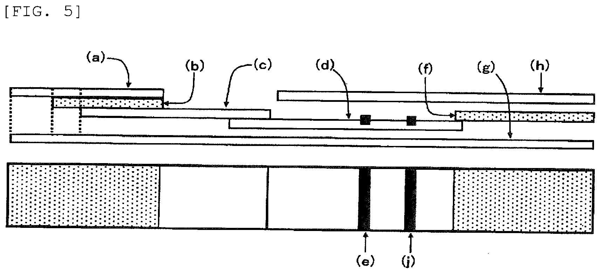

Fig. 5 shows a schematic structure of an exemplary immunochromatographic test strip of the present invention.

### DESCRIPTION OF EMBODIMENTS

### (Analyte)

An analyte of the present invention is oncofetal fibronectin (fFN).

### (Sample)

In the present invention, the "sample" may be any one as long as it may include fFN which is an analyte, and the sample may be exemplified by a cervicovaginal fluid, a cervical fluid, a vaginal fluid, etc.

A component separated and fractionated from the sample by means of centrifugation, filtration, purification, etc., a component extracted with an organic solvent, etc., a component solubilized by a surfactant, etc., a component diluted with a buffer solution, etc., and a component modified/altered by chemical reaction, etc. are also included in the sample of the present invention.

In the present specification, the "sample" may also be called "specimen", and they are used as synonyms, and the "sample" is a liquid (fluid) when it is added and spread to the solid phase of the immunochromatography of the present invention.

### (Specific Surfactant)

The specific surfactant of the present invention is polyoxyethylene alkyl ether represented by the following Formula (1):

R-O-(CH₂CH₂O)ₘH (1)

wherein m is 4 to 30, and R represents an alkyl group.

The polyoxyethylene alkyl ether functions to inhibit cross-reactivity between plasma FN in the sample and an anti-fFN antibody in the method of detecting fFN using immunochromatography.

In the polyoxyethylene alkyl ether represented by Formula (1) of the present invention, the carbon number of R (alkyl group) which is a hydrophobic group thereof may be 8 to 20. Specifically, the hydrophobic group may be, for example, a lauryl group (12), a cetyl group (16), a stearyl group (18), an oleyl group (18) or the like (the numerical value in parenthesis is the carbon number of the alkyl group). In addition, m, that is, the number of ethylene oxide groups in the above formula, which is a hydrophilic group of polyoxyethylene alkyl ether, is preferably 4 or more and 30 or less, more preferably 10 or more and 30 or less, and particularly preferably 12 or more and 25 or less. Such polyoxyethylene alkyl ether may be exemplified by polyoxyethylene (12) lauryl ether, polyoxyethylene (23) lauryl ether, polyoxyethylene (13) oleyl ether or the like. Polyoxyethylene (23) lauryl ether is particularly preferred. As the polyoxyethylene alkyl ether, commercially available EMULGEN (trade name), Brij (trademark), NIKKOL (trade name), or EMALEX (trade name) may be used.

With respect to the polyoxyethylene alkyl ether represented by Formula (1), its function of suppressing the cross-reactivity between the anti-fFN antibody and FN in the case where fFN and FN which is an antigen similar to fFN coexist in a sample is not known.

As a use mode of the specific surfactant of the present invention, antigens similar to fFN (e.g., plasma FN) in a sample are preferably allowed to bringing into contact with the surfactant before the sample brings into contact with a detection antibody, or the surfactant may be generally included in a specimen extract solution (also referred to as a specimen dilution solution) or a spreading solution. Of them, it is preferable that the surfactant is included in the specimen extraction solution.

### (Specimen Extraction Solution)

In the present specification, the specimen extraction solution is used as a synonym for the specimen dilution solution.

The specimen extraction solution may be used in need of extracting and diluting a sample according to the concentration of the analyte in the sample. The specimen extraction solution may be an extraction solution of any composition, as long as it does not significantly inhibit antigen-antibody reaction, or conversely, does not significantly facilitate the reaction resulting in excessive aggregation of the labeling substance, which causes failure of spreading by capillarity, or does not completely prevent antigen concentration-dependent signal detection of the antigen-antibody reaction.

The specimen extraction solution having these actions may be, for example, purified water, a physiological saline solution, or a low-concentration buffer solution such as 10 mmol/L to 20 mmol/L of a phosphate buffer solution, 10 mmol/L to 20 mmol/L of a Tris-HCl buffer solution, or 10 mmol/L to 20 mmol/L of a Bis-Tris buffer solution.

As described above, a preferred use mode is that the specimen extraction solution of the present invention includes the specific surfactant of the present invention.

A concentration of the surfactant of the present invention in the specimen extraction solution may be any concentration as long as it may suppress cross-reactivity other than the reaction between fFN and a specific antibody thereto, and a lower limit of the concentration of the surfactant in the solution may be more than 0.2% (W/V), 0.25% (W/V), 0.3% (W/V), 0.4% (W/V), or 0.5% (W/V) . In addition, an upper limit thereof may be less than 1.0% (W/V), 0.95% (W/V), 0.9% (W/V), 0.80(W/V), 0.7%(W/V), or 0.6%(W/V). A range of the concentration is preferably more than 0.2%(W/V) and less than 1.0%(W/V). In addition, a combination of any one of the upper limits and any one of the lower limits may be mentioned.

### (Spreading Solution)

In the present invention, the spreading solution refers to a solution which spreads on the immunochromatographic test strip. Therefore, in some cases, the specimen extraction solution and the specimen dilution solution may also serve as the spreading solution, but in this specification, a solution not containing the specimen and aiming only for spreading is particularly distinguished as the spreading solution. In this case, detection occurs in the step of supplying the spreading solution after supplying only the specimen or a liquid obtained by diluting the specimen with a small amount of the specimen dilution solution to the immunochromatographic test strip. As the spreading solution of the present invention, purified water, a physiological saline solution, or a low-concentration buffer solution as used in the above-mentioned specimen extraction solution may be used, and a concentration of the surfactant of the present invention in the spreading solution is also the same as in the specimen extraction solution.

### (Immunochromatographic Test Strip)

The immunochromatographic test strip of the present invention is a membrane consisting of a porous body equipped with at least a "sample-supplying portion", a "spreading portion", and a "detecting portion", and has a structure in which a labeled antibody against fFN which is an analyte is retained at a spreading start part of the spreading portion in a dissoluble manner such that the labeled antibody passes through the spreading portion and reaches the detecting portion after contact with a sample, while an antibody against fFN is immobilized at a part of the spreading portion to constitute the detecting portion.

An example of embodying these elements may be a test strip including a sample pad playing a role of a sample-supplying portion, a conjugate pad having a labeled antibody against fFN retained in a dissoluble manner and playing a role of the spreading portion, and an insoluble membrane having an immobilized antibody immobilized at a part thereof and playing a role of the spreading portion and the detecting portion. That is, a typical immunochromatographic test strip of the present invention has the following configuration:
(1) a sample pad to which a sample is supplied;
(2) a conjugate pad which is disposed in the downstream of the sample pad and retains a conjugate in a dissoluble manner, the conjugate having a first antibody sensitized on a colloidal gold surface; and
(3) an insoluble membrane to which a second antibody binding to a complex of the conjugate and fFN is immobilized, and the membrane being disposed in the downstream of the conjugate pad,
wherein the sample pad, the conjugate pad, and the insoluble membrane may constitute respective different carriers, or two of the elements may constitute one carrier, and any mode may be taken as long as the sample pad, the conjugate pad, and the insoluble membrane are arranged in this order from the upstream toward the downstream of the flow of the sample.

The immunochromatographic test strip may be a strip further having any one or more of an absorption pad and a third pad disposed and mounted along with the above constituents. The test strip is usually disposed on a solid phase support such as a plastic adhesive sheet. It is obvious that the solid phase support is made of a material not hindering the capillary flow of the sample and that an adhesive component is made of a material not hindering the capillary flow of the sample. Further, the test strip may be laminated with a polyester film, etc., for the purpose of increasing the mechanical strength of the antibody-immobilized membrane and preventing evaporation (drying) of water during an assay.

### (Label)

As a "label" of a labeled antibody used in the present invention, visualizable microparticles such as metal colloid particles (colloidal gold, etc.) and colored particles (colored latex particles, etc.), magnetic particles, fluorescent particles, enzymes such as horseradish peroxidase, alkaline phosphatase, and β-D-galactosidase, etc., radioisotopes such as iodine-125, etc., luminescent substances such as acridinium compounds, luminol, etc., fluorescent substances such as fluorescein isothiocyanate, europium (III) chelate, etc. are known. The metal colloid and colored latex are preferred. As a method of labeling an antibody, etc. (i.e., a method of introducing and binding a labeling substance), any known method may be used without limitation.

Colloidal gold may be any colloidal gold as long as it is able to form a conjugate through sensitization with (immobilization of) an antibody and to serve as a label in a method of detecting a target object (antigen) in a sample through contact with the sample.

As the colloidal gold, colloidal platinum as well as colloidal gold is included in the colloidal gold of the present invention.

A particle diameter of colloidal gold particles is known to significantly affect the detection sensitivity, and for example, when the colloidal gold particles are retained and used in an immunochromatographic test strip, the particle diameter of colloidal gold particles is preferably 20 nm to 60 nm, more preferably 40 nm to 60 nm, and particularly preferably 45 nm to 55 nm. The colloidal gold may be manufactured by a generally known method, for example, by dropping and stirring a trisodium citrate aqueous solution or a triammonium citrate aqueous solution in a heated tetrachloroauric(III) acid aqueous solution.

### (Sensitization of Antibody with Colloidal Gold)

Immobilization of antibody against an analyte onto colloidal gold is generally achieved by physical adsorption. In this regard, a concentration of the antibody is preferably adjusted to a concentration of 1 µg/mL to 5 µg/mL buffer solution. A type and pH of the buffer solution are preferably 2 mmol/L to 10 mmol/L Tris buffer solution (pH 7 to pH 9) . However, another buffer solution may be used without any limitation thereto. In this description, the above-described colloidal gold, onto which an antibody against an analyte or a control antibody (or antigen) is immobilized, is called a "conjugate".

### (Blocking)

The conjugate of the present invention may be blocked by a blocking agent in a region not bound with an antibody of a colloidal gold surface.

The blocking agent of the colloidal gold conjugate is generally a component derived from a living organism or a component derived from a non-living organism, and the component derived from a living organism may be any component as long as it is derived from the living organism and has a blocking effect, and for example, the component includes an animal protein or an animal protein-derived peptide. Specifically, the component may include bovine serum albumin (BSA), Blocking Peptide Fragment (manufactured by TOYOBO) derived from microorganisms, NEO PROTEIN SAVER (manufactured by TOYOBO) derived from silk protein (hydrolysate of sericin), StartingBlock™ (PBS) Blocking Buffer (manufactured by PIERCE), StabilCoat™ (manufactured by SurModics), and casein.

A concentration of the component derived from the living organism may be appropriately determined depending on the component to be used. For example, an antibody solution is added to and mixed with a colloidal gold solution adjusted to 1 OD/mL, and then the component derived from the living organism is added to the mixed solution at a final concentration within a range of 0.1% to 10% for blocking, and more preferably used within a range of 0.2% to 5%.

Alternatively, a mixture of both a component derived from a non-living organism and a component derived from a living organism may be used as a blocking agent of colloidal gold.

The term "detection" or "measurement", as used herein, must be construed in the broadest sense including verification and/or quantification of the presence of the analyte and must not be construed in a limited manner in any sense.

### (Sample Pad)

In the present invention, a "sample pad" is a part that serves as a sample-supplying portion receiving a sample, and is shaped into a pad to absorb a liquid sample, and the sample pad may include any material and form allowing the passage of a liquid and the analyte component.

The sample pad may contain the buffer solution. In this case, the buffer solution may be contained at least in a part or entirety of the sample pad.

Specific examples of materials suitable for the sample pad may include, but are not limited to, a glass fiber, an acrylic fiber, a hydrophilic polyethylene material, a dry paper, a paper pulp, a fabric, etc. A glass fiber pad is preferably used. The sample pad may additionally be given a function of a conjugate pad described later. The sample pad may contain a commonly used blocking reagent as needed within a range not affecting the reaction system and not departing from the object of the present invention.

### (Conjugate Pad)

The "conjugate pad", as used herein, refers to a pad which is obtained by impregnating a material suitable for the conjugate pad described later with a detection reagent specifically reactive with the analyte, followed by drying. The conjugate pad has a function of allowing the detection reagent and the analyte to form a complex when the sample passes through the conjugate pad. The conjugate pad may by itself be disposed in contact with an antibody-immobilized membrane. Alternatively, the conjugate pad may be disposed in contact with the sample pad so as to receive the sample which has passed through the sample pad by a capillary flow and then transfer the sample by a capillary flow to another pad (hereinafter, also referred to as a "third pad") in contact with the surface different from the contact surface with the sample pad. The selection of one or more parts of the sample pad and the conjugate pad and how the selected parts are disposed on the antibody-immobilized membrane may be appropriately changed.

The conjugate pad may also contain the buffer solution. In this case, the buffer solution is contained at least in a part including the upstream side from a part to which the conjugate is immobilized, and may be contained in entirety of the conjugated pad.

Materials suitable for the conjugate pad may include, but are not limited to, paper, a cellulose mixture, nitrocellulose, polyester, an acrylonitrile copolymer, a glass fiber, and a nonwoven fiber such as rayon. A glass fiber pad is preferably used.

The conjugate pad may contain, as needed, a "control reagent" for ensuring reliability of immunochromatography, for example, an antibody labeled with a label and not reactive with the sample component, or a highly antigenic protein such as KLH (keyhole limpet hemocyanin) labeled with a label. These control reagents are components (substances) having no possibility of being present in the sample and may appropriately be selected.

### (Third Pad)

In the present invention, a third pad may be disposed for the purpose of removing components unnecessary for detection of the analyte from the reaction components of the sample and the detection reagent so that components necessary for reaction may smoothly spread in an insoluble membrane to which an antibody is immobilized.

For example, blood cells, insoluble blood cell fractures, etc. are preferably removed as the components unnecessary for detection. The third pad may also be given an additional effect of preliminarily removing aggregates, which are grown to a size which prevents the movement to and the smooth spread in the antibody-immobilized membrane, out of aggregates generated by antigen-antibody reactions. The third pad may include any material and form allowing the passage of the components of the liquid and the analyte component.

Specific examples include, but are not limited to, a glass fiber, an acrylic fiber, a hydrophilic polyethylene material, a dry paper, a paper pulp, a fabric, etc.

### (Immobilization of Antibody to Insoluble Membrane)

In the immunochromatographic reagent of the present invention, immobilization of the antibody against fFN which is an analyte (anti-fFN antibody) to the insoluble membrane may be performed by a generally well-known method. For example, in the case of the flow-through format, the anti-fFN antibody is prepared at a predetermined concentration and a given amount of the solution thereof is applied to the insoluble membrane in a shape of dot or a specific symbol such as "+". In this case, to ensure reliability of immunochromatography, a "control line" is generally formed by immobilizing a protein or a compound capable of binding to the conjugate to a position different from the anti-fFN antibody. The "control line" may be formed by immobilizing the antibody against the control reagent to a position different from the anti-fFN antibody.

In the case of a lateral-flow format, the antibody is prepared at a predetermined concentration and the solution thereof is applied to an insoluble membrane in a line shape by using a device having a mechanism capable of horizontally moving while discharging the solution from a nozzle at a constant rate. In this regard, a concentration of the antibody is preferably 0.1 mg/mL to 5 mg/mL, and more preferably 0.5 mg/mL to 3 mg/mL. An immobilized amount of the antibody on the insoluble membrane may be optimized by adjusting the application amount dropped onto the insoluble membrane in the case of the flow-through format, and optimized by adjusting the discharge rate from the nozzle of the device in the case of the lateral-flow format. Particularly, in the case of the lateral-flow format, 0.5 µL/cm to 2 µL/cm is preferable. In the present invention, a "flow-through membrane assay" refers to a format in which the sample liquid, etc. spreads to perpendicularly pass through the insoluble membrane, and a "lateral-flow membrane assay" refers to a format in which the sample liquid, etc. spreads to move in parallel with the insoluble membrane.

In the present invention, the application position of an antibody against a target object to the insoluble membrane may be placed such that the detection reagent spreads from the conjugate pad by capillarity and sequentially passes through the lines to which the respective antibodies are applied in the case of the lateral-flow format. Preferably, the line formed by applying the antibody against the analyte is preferably located upstream while the line formed by applying a control antibody is located downstream thereof. In this case, a sufficient distance is preferably placed between the respective lines such that signals of labels may be detected. In the case of the flow-through format, the position of application of the antibody against the target object may be placed such that signals of labels may be detected.

The antibody solution applied to the insoluble membrane may normally be prepared by using a predetermined buffer solution. A type of the buffer solution may include commonly used buffer solutions such as a phosphate buffer solution, a Tris buffer solution, a Good's buffer solution, etc. pH of the buffer solution is preferably in a range of 6.0 to 11 and may be appropriately determined depending on properties of the antibody to be used. For example, a buffer solution of pH 7.2 is usable for an anti-fFN monoclonal antibody described later.

The buffer solution may contain a salt such as NaCl, etc. , a stabilizer such as sucrose or a preservative, and antiseptic such as ProClin, etc. The salt may include those contained for adjusting ionic strength, such as NaCl, as well as those added at the step of adjusting pH of the buffer solution, such as sodium hydroxide. After the antibody is immobilized to the insoluble membrane, blocking may be performed by coating a portion other than the antibody-immobilized parts with a commonly used blocking agent in a solution or in a vapor state. In this description, the insoluble membrane having the antibody immobilized as described above is also referred to as an "antibody-immobilized membrane".

### (Insoluble Membrane)

In the present invention, the insoluble membrane (hereinafter, also simply referred to as a membrane) may be made of any material. For example, the materials may include, but are not limited to, polyethylene, polyethylene terephthalate, nylons, glass, polysaccharide such as cellulose and cellulose derivatives, or ceramics. Specifically, the materials may include glass fiber filter papers and cellulose filter papers available from Merck & Co. , Inc., Toyo Roshi kaisha, Ltd., Whatman, Inc., etc. A pore size and structure of the insoluble membrane may be appropriately selected, thereby controlling a flow speed of an immune complex of a colloidal gold-labeled antibody and a target object through the membrane. The amount of the labeled antibody binding to the antibody immobilized to the membrane may be adjusted by controlling the flow speed through the membrane, and therefore, the pore size and the structure of the membrane are preferably optimized in consideration of combinations with the other constituent materials of the immunochromatographic test strip of the present invention.

### (Absorption Pad)

In the present invention, the absorption pad refers to a liquid-absorbing part that absorbs the sample which has moved on and passed through the insoluble membrane to control the spread of the sample. The absorption pad may be disposed at the most downstream portion of the strip configuration in the lateral-flow format, and may be disposed on, for example, the lower portion of the antibody-immobilized membrane in the flow-through format. The absorption pad may be made of, for example, filter paper, but is not limited thereto.

### (Detection Device)

The immunochromatographic test strip of the present invention may be used after being housed/mounted in an appropriate container (housing) in consideration of a size of the strip, an addition method and position of the sample, an immobilization position of antibody on the antibody-immobilized membrane, a signal detection method, etc., and the immunochromatographic test strip in such a housed/mounted state is referred to as a "device".

### (Others)

In this description, the "insoluble membrane" is also referred to as a "solid phase", and allowing the insoluble membrane to physically or chemically support antigens or antibodies or a state of allowing the support may be expressed as "immobilization", "immobilized", "solid-phased", "sensitization", or "adsorption".

### (Antibody Used in the Present Invention)

The antibody against the analyte used in the present invention is not limited in any way to a preparation method as long as the antibody is specifically reactive to the analyte, and may be a polyclonal antibody or a monoclonal antibody. A hybridoma producing the corresponding antibody may be generally prepared by cell fusion between spleen cells of an animal immunized with the analyte as an immunogen and myeloma cells from the same species in accordance with the method of Kohler and Milstein (see Nature, Vol. 256, p. 495, 1975).

When antibodies used in a measurement method of detecting the analyte through formation of so-called sandwich are monoclonal antibodies, a relationship between an antibody labeled with a labeling substance (first antibody) included in the conjugate and an insoluble membrane-immobilized antibody (second antibody) to be immobilized in the spreading portion is such that the epitope of the second antibody is different from that of the first antibody. It is preferable that any one of the first and second antibodies is an anti-fFN antibody, and the other is an antibody binding to fFN. It is more preferable that the first antibody is the antibody binding to fFN, and the second antibody is the anti-fFN antibody. The antibody binding to fFN may be any antibody binding to fFN, and any antibody binding to plasma FN which is an antigen similar to fFN, and with which the sample may be contaminated.

In Example 1 described later, fFN is supposed as an analyte protein, and plasma FN is supposed as an antigen similar thereto. An anti-fFN monoclonal antibody that specifically binds to fFN and does not bind to plasma FN (hereinafter, referred to as an anti-fFN monoclonal antibody) and an anti-fibronectin antibody that binds to both fFN and plasma FN (hereinafter, referred to as an anti-FN antibody) are used.

fFN and plasma FN are glycoproteins that have the same amino acid sequence, but differ only in the sugar chain content. Here, in the collection of a vaginal secretion, etc., which is a specimen containing fFN, of a pregnant woman, there is a possibility of blood contamination, and therefore, the specimen contains both plasma FN and fFN. It is difficult to obtain two or more kinds of the fFN-specific antibodies because such antibodies recognize the difference of the sugar chain. Therefore, when the antibodies are used in a sandwich immunochromatography, it may be necessary to construct an assay system with two kinds of antibodies, in which one is an fFN monoclonal antibody and the other is an anti-FN monoclonal antibody.

The anti-fFN monoclonal antibody used in the present invention is a monoclonal antibody, which is obtained by synthesizing a glycopeptide containing a sugar chain binding site peculiar to fFN, binding it to transferrin or OVA by a carbodiimide method, and performing footpad immunization and subcutaneous immunization with the resulting product as an immunogen, and specifically reacts with fFN. Further, the anti-FN monoclonal antibody used in the present invention is a monoclonal antibody, which is obtained by similarly performing immunization with a full-length protein of plasma FN as an immunogen, and reacts with any one of fFN and plasma FN.

The present invention is not limited thereto, and a commercially available anti-FN monoclonal antibody and anti-fFN monoclonal antibody may also be used. Examples of the commercially available anti-FN monoclonal antibody may include Anti-Human Fibronectin, Monoclonal (Clone FN12-8), Anti-Human Fibronectin, Monoclonal (Clone FN30-8) (manufactured by Takara Bio, Inc.) (It is noted that monoclonal antibodies may be denoted by clone names of hybridomas producing the respective antibodies for convenience. The same applies hereinafter).

When fFN is detected, any one of the first antibody and the second antibody is preferably the anti-fFN monoclonal antibody, and the other is preferably the anti-FN monoclonal antibody, and more preferably, the first antibody is the anti-FN monoclonal antibody, and the second antibody is the anti-fFN monoclonal antibody.

### (Preparation Example of Anti-fFN Monoclonal Antibody and Anti-FN Monoclonal Antibody)

The anti-fFN monoclonal antibody (Clone # 90204) used in the following test was obtained by immunizing mice with fFN peptide fragment-bound OVA or fFN peptide fragment-bound transferrin, and the anti-FN monoclonal antibody (Clone # 90412) was obtained by immunizing mice with a full-length protein of plasma fibronectin according to a method which is generally performed by those skilled in the art in order to prepare monoclonal antibodies (method of Kohler and Milstein, (Nature, Vol. 256, p. 495, 1975)).

### (Measurement)

A method of quantifying signals derived from colloidal gold may be performed in accordance with a known method, and absorbance or reflected light intensity may be measured. Alternatively, the changes in absorbance or reflected light intensity may be extrapolated to a calibration curve of samples with known concentrations to measure the concentration of the target object.

### (Kit)

A combination of the immunochromatographic test strip, and any one or more of the specimen extraction solution, and an instruction manual may be used as a kit. In this case, the surfactant of the present invention may be contained in any one or more of the specimen extraction solution, the specimen dilution solution, the spreading solution.

### (Detection Method Using Immunochromatography)

The detection method using immunochromatography of the present invention is a method comprising at least the following steps of (A) to (C), and hereinbelow, a typical detection method using fFN as an analyte and plasma FN as a similar antigen will be described:
(A) supplying a surfactant of the present invention and a specimen extraction solution containing a sample to a sample-supplying portion of a test strip comprising a membrane consisting of a porous body equipped with at least the sample-supplying portion, a spreading portion, and a detecting portion, wherein a colloidal gold-labeled anti-FN antibody (conjugate) is retained in a part of the spreading portion in a dissoluble manner, and the detecting portion in which an anti-fFN antibody is immobilized is provided in a part of the spreading portion on the downstream side relative to the conjugate-retaining part;
(B) bringing the sample (specimen) into contact with the conjugate; and
(C) detecting a complex of fFN in the sample and the colloidal gold-labeled anti-FN antibody (conjugate) obtained in step (B) in the detecting portion to which the anti-fFN antibody is immobilized.

### [EXAMPLE]

### [Test Example 1] Production of Immunochromatographic Test Strip of the Present Invention

### 1. Preparation of Colloidal Gold-Labeled Anti-FN Antibody Solution

The preparation was performed by the following procedure.
(i) A 50-nm colloidal gold stock solution was diluted with purified water to 1.0 OD, and adjusted to pH 8.5 with a 0.2 M potassium carbonate aqueous solution.
(ii) Anti-plasma FN mouse monoclonal antibody (clone # 90412) was diluted with 5 mM boric acid to be 80 µg/mL.
(iii) BSA was dissolved in purified water so as to be a 10% solution.
(iv) 1 mL of (ii) was added to 20 mL of (i) under stirring, followed by stirring for 10 minutes.
(v) 2 mL of (iii) was added to (iv) under stirring, followed by stirring for 5 minutes.
(vi) (v) was centrifuged at 10°C and 10,000 rpm for 45 minutes, the supernatant was aspirated, and the precipitate was suspended with 1 mL of a conjugate-diluting solution (Buffer Conjugate Dilution, SCRIPPS) to obtain a colloidal gold-labeled anti-FN antibody solution (Anti-FN antibody conjugate).
(vii) (vi) was heated at 45°C for 18 hours.

### 2. Production of Conjugate Pad

The colloidal gold-labeled anti-FN antibody solution prepared in 1 was mixed with a 20 mM aqueous solution of monosodium phosphate containing 2.0% BSA and a 2.4% lactose solution so as to be 3.75 OD/mL, to prepare a conjugate solution (pH 7). The conjugate solution was impregnated into a glass fiber pad on a cookie sheet at a rate of 66.93 µL/cm², and dried in a dry oven at 70°C for 45 minutes, and this pad was used as a conjugate pad.

### 3. Production of Sample Pad

A 20 mM aqueous solution of monosodium phosphate (pH 7) was impregnated into a glass fiber pad on a cookie sheet at a rate of 62.99 µL/cm², and dried in a dry oven at 70°C for 45 minutes, and this pad was used as a sample pad.

### 4. Preparation of Anti-fFN Antibody and Anti-mouse IgG Antibody Membrane Application Solutions

### (i) Preparation of anti-fFN antibody membrane application solution

An anti-fFN mouse monoclonal antibody (clone # 90204) was diluted with a 10 mM phosphate buffer (pH 7.2) containing 2.5% sucrose so that the antibody concentration became 2.0 mg/mL, and this solution was used as an anti-fFN antibody membrane application solution.

### (ii) Preparation of anti-mouse IgG antibody membrane application solution

As in (i), an anti-mouse IgG goat polyclonal antibody (Jackson Immuno Research Laboratories) was diluted with a 10 mM phosphate buffer (pH 7.2) containing 2.5% sucrose so that the antibody concentration became 0.5 mg/mL, and this solution was used as an anti-mouse IgG goat polyclonal antibody membrane application solution.

### 5. Production of Antibody-Immobilized Membrane

An immunochromatography dispenser XYZ3000 (BIO DOT) was used to apply the anti-fFN antibody membrane application solution and the anti-mouse IgG antibody membrane application solution at a position of 11 mm and 15 mm distant from the lower end of a nitrocellulose membrane (Hi-Flow Plus HF180 (Merck Millipore)) at 1.0 µL/cm, respectively and dried in a dry oven at 70°C for 45 minutes to produce an antibody-immobilized membrane.

### 6. Production of Test Device

To a plastic adhesive sheet (g), the antibody-immobilized membrane (d) was affixed, antibody-application sites were arranged in the order of the anti-fFN antibody (e) on the upstream side of spread, and then the anti-mouse IgG antibody (j) as a control reagent, and a third pad (c) was mounted. Next, the above-prepared conjugate pad (b) was placed and mounted, and the above-prepared sample pad (a) was placed and mounted so as to overlap with this conjugate pad, while the absorption pad (f) was placed and mounted on the end of the other side. A transparent plastic film (h) was placed on the surface of the antibody-immobilized membrane and the absorption pad, and an immunochromatographic test strip was produced by cutting a structure having the constituting elements overlapped with each other as described above.

The test strip was housed/mounted in a dedicated plastic housing having a sample-addition window and a detection window (DKVIN (Syn corporation) (not shown in FIG. 6)) at the time of an assay to implement a form of an immunochromatographic test device. FIG. 6 shows a schematic structure of the immunochromatographic test strip.

### [Example 1] Relationship between kinds of surfactant added to specimen extraction solution and FN cross-reactivity

A kind of a surfactant added to a specimen extraction solution was changed by using the test device produced above, and their effects on the cross-reactivity of plasma FN were confirmed. Unless otherwise specified for the concentration of the non-ionic surfactant, % represents %(w/v).

### 1. Test Method

### (1) Preparation of Specimen

A plasma pool of normal people was diluted with the following fFN specimen extraction solutions to prepare specimens of 0.3% plasma.

### <Composition of fFN specimen extraction solution>

10 mM PBS, 1% BSA, 1 mM EDTA-2Na, nonionic surfactants (0.1%, 0.5%) shown in Table 1

**[Table 1]**

| | Nonionic surfactant |
|---|---|
| 1 | TritonX-114 (Prior art) |
| 2 | TritonX-100 |
| 3 | n-Heptyl-*β*-D-thioglucoside |
| 4 | n-Octyl-*β*-D-glucoside |
| 5 | Tween20 |
| 6 | Brij35 (Brij is a trademark) |

### (2) Measurement

120 µL of each specimen was dropped to the test device, and after 10 minutes, the reflection absorbance of the test line and the control line was measured with RAPIDPIA ((registered trademark) Sekisui Medical Co., Ltd.) and software (C10066).

### 2. Test Results

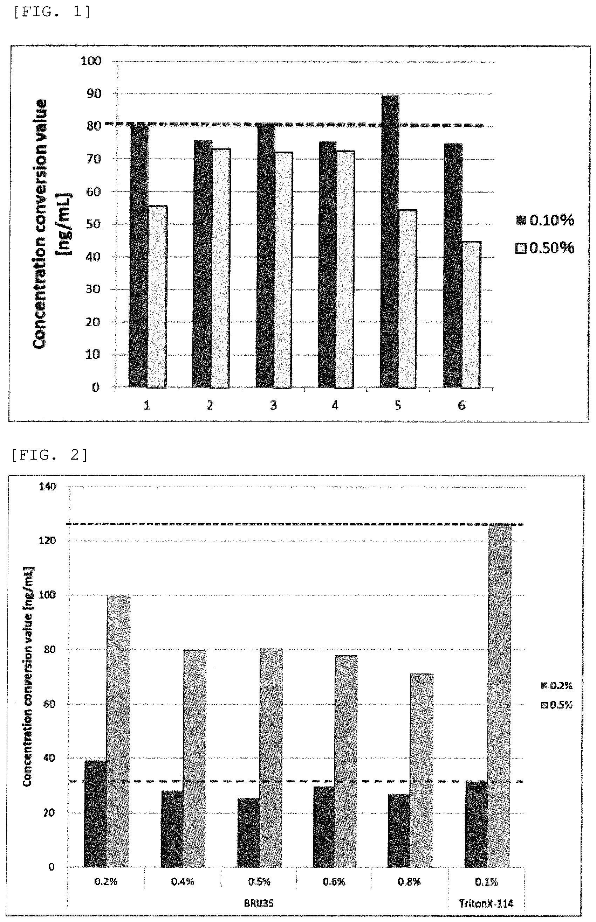

Results are shown in Fig. 1. In the figure, the result of the cross-reactivity with plasma FN at the time of adding 0.1% Triton X-114 is indicated by a dotted line.

Among the nonionic surfactants added to the specimen extraction solution, Brij 35 most greatly inhibited the cross-reactivity with plasma FN when 0.1% and 0.5% thereof were added. Therefore, in the next test, the addition concentration of Brij 35 was changed and its inhibitory effect on cross-reactivity was investigated.

### [Example 2] Relationship between addition concentration of Brij 35 and FN cross-reactivity

### 1. Test Method

A test was performed in the same manner as in Example 1, except that a plasma pool of normal people was diluted with fFN specimen extraction solutions containing 0.2%, 0.4%, 0.5%, 0.6%, or 0.8% of Brij 35 as a nonionic surfactant to adjust the concentration of plasma to 0.2% and 0.5%. As Comparative Example, a Triton X-114-added fFN specimen extraction solution (prior art) was also tested.

### 2. Test Results

Results are shown in Fig. 2.

When Brij 35 was added in the concentration range of 0.4% to 0.8%, the cross-reactivity with plasma FN was inhibited, as compared with addition of 0.1% Triton X-114 which is a prior art.

### [Example 3] Relationship between chain length of Brij and FN cross-reactivity

### 1. Test Method

A test was performed in the same manner as in Example 1, except that a plasma pool of normal people was diluted with fFN specimen extraction solutions containing 0.1% or 0.5% of the nonionic surfactants shown in Table 2 to adjust the concentration of plasma to 0.2% and 0.3%. As Comparative Example, a 0.1% or 0.5% Triton X-114-added fFN specimen extraction solution (prior art) was also tested.

**[Table 2]**

| Nonionic surfactant | | R-O-(CH₂CH₂O) ₘH | |
|---|---|---|---|
| Trade name | Common name | Value of m | R |
| TritonX-114 (Comparative Example) | - | - | - |
| Brij35 | Polyoxyethylene (23) lauryl ether | 23 | Lauryl |
| BrijO20 | Polyoxyethylene(20) oleyl ether | 20 | Oleyl |
| BrijS100 | Polyoxyethylene(100) stearyl ether | 100 | Stearyl |
| Brij 93 | Polyoxyethylene(2) oleyl ether | 2 | Oleyl |

### 2. Test Results

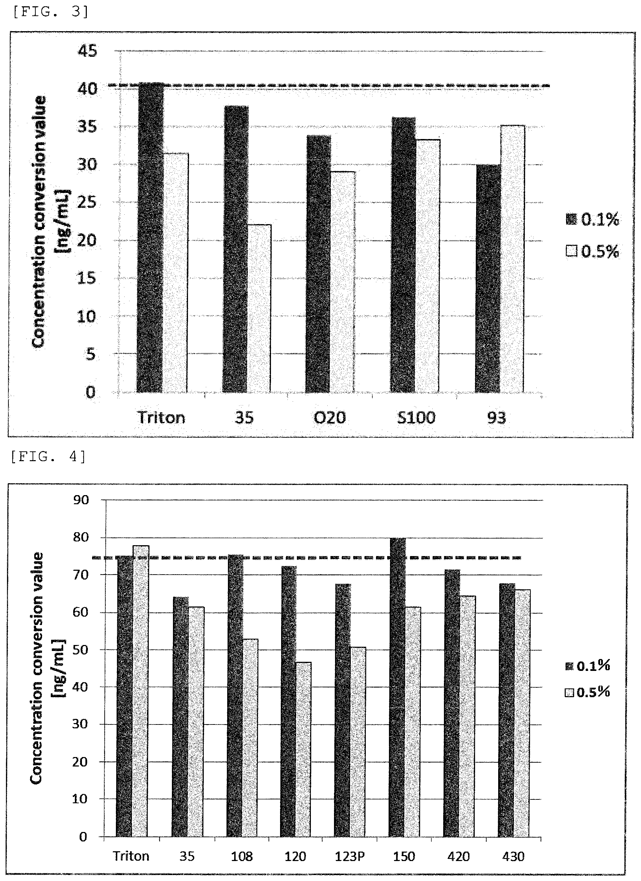

Results are shown in Fig. 3.

Both concentrations of Brij 35 (m=23) and Brij 020 (m=20) inhibited cross-reactivity with plasma fibronectin to exhibit high inhibitory effect, as compared with the same concentrations of Triton X-114. In contrast, 0.5% of Brij S100 (m=100) and Brij 93 (m=2) failed to inhibit the cross-reactivity with plasma fibronectin to exhibit low inhibitory effect, as compared with the same concentration of Triton X-114.

### [Example 4] Relationship between chain length of EMULGEN and FN cross-reactivity

### 1. Test Method

A test was performed in the same manner as in Example 3, except that a plasma pool of normal people was diluted with fFN specimen extraction solutions containing the nonionic surfactants shown in Table 3 to adjust the concentration of plasma to 0.3%.

**[Table 3]**

| Nonionic surfactant | | R-O-(CH₂CH₂O)ₘH | |
|---|---|---|---|
| Trade name | Common name | Value of m | R |
| TritonX-114 (Comparative Example) | - | - | - |
| Brij35 | Polyoxyethylene(23) lauryl ether | 23 | Lauryl |
| EMULGEN 108 | Polyethylene (6) lauryl ether | 6 | Lauryl |
| EMULGEN 120 | Polyoxyethylent(12) lauryl ether | 12 | Lauryl |
| EMULGEN 123P | Polyoxyethylene(23) lauryl ether | 23 | Lauryl |
| EMULGEN 150 | Polyoxyethylene(47) lauryl ether | 47 | Lauryl |
| EMULGEN 420 | Polyoxyethylene(13) oleyl ether | 13 | Oleyl |
| EMULGEN 430 | Polyoxyethylene(30) oleyl ether | 30 | Oleyl |

### 2. Test Results

Results are shown in Fig. 4.

Both concentrations of EMULGEN 108 (m=6), EMULGEN 120 (m=12), EMULGEN 123P (m=23), EMULGEN 420 (m=20), and EMULGEN 430 (m=30) inhibited cross-reactivity with plasma fibronectin to exhibit high inhibitory effect, as compared with the same concentrations of Triton X-114. In contrast, 0.5% of EMULGEN 150 (m=47) inhibited the cross-reactivity with plasma fibronectin, as compared with the same concentration of Triton X-114.

### INDUSTRIAL APPLICABILITY

According to the present invention, in a method of detecting oncofetal fibronectin (fFN) using immunochromatography, even when fFN is contaminated with blood, measurement errors or false positive reactions hardly occur and fFN may be accurately detected. Accordingly, it is possible to provide a detection reagent for threatened premature labor using immunochromatography which is frequently used in POCT.

### REFERENCE SIGNS LIST

(a) Sample pad
(b) Conjugate pad
(c) Third pad
(d) Antibody-immobilized membrane
(e) anti-fFN antibody
(f) Absorption pad
(g) Plastic adhesive sheet
(h) Plastic film
(j) Control antibody

## Claims

1. A detection method of oncofetal fibronectin using immunochromatography, comprising steps (A) to (C):
(A) supplying a sample possibly containing oncofetal fibronectin and fibronectins other than oncofetal fibronectin and a liquid composition containing a non-ionic surfactant represented by Formula (1) to a sample-supplying portion of the following test strip;
the test strip comprising a membrane consisting of a porous body equipped with at least the sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, and wherein any one of the first antibody and the second antibody is an anti-oncofetal fibronectin antibody and the other is an antibody binding to oncofetal fibronectin;
(B) bringing the sample into contact with the conjugate; and
(C) detecting a complex of oncofetal fibronectin in the sample and the conjugate in the detecting portion:
R-O-(CH₂CH₂O)ₘH (1)
wherein m is 4 to 30, and R represents an alkyl group.

2. The detection method of claim 1, wherein a concentration of the surfactant in the liquid composition is in the range of more than 0.2%(W/V) and less than 1.0%(W/V).

3. A method of reducing cross-reaction between oncofetal fibronectin and fibronectins other than oncofetal fibronectin, in a method of detecting oncofetal fibronectin in a sample using an immunochromatographic test strip,
the method of reducing cross-reaction comprising the following steps:
(A) supplying a sample possibly containing oncofetal fibronectin and fibronectins other than oncofetal fibronectin and a liquid composition containing a non-ionic surfactant represented by Formula (1) to a sample-supplying portion of the following test strip;
the test strip comprising a membrane consisting of a porous body equipped with at least the sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, and wherein any one of the first antibody and the second antibody is an anti-oncofetal fibronectin antibody and the other is an antibody binding to oncofetal fibronectin;
(B) bringing the sample into contact with the conjugate; and
(C) detecting a complex of oncofetal fibronectin in the sample and the conjugate in the detecting portion:
R-O-(CH₂CH₂O)ₘH (1)
wherein m is 4 to 30, and R represents an alkyl group.

4. The method of reducing cross-reaction of claim 3, wherein a concentration of the surfactant in the liquid composition is in the range of more than 0.2% (W/V) and less than 1.0% (W/V) .

5. An immunochromatographic detection kit for oncofetal fibronectin, comprising the following components (1) and (2) :
(1) an immunochromatographic test strip comprising a membrane consisting of a porous body equipped with at least a sample-supplying portion, a spreading portion, and a detecting portion, wherein a conjugate containing a first antibody labeled with a labeling substance is retained in a part of the spreading portion in a dissoluble manner, and the detecting portion in which a second antibody is immobilized is provided in a part of the spreading portion on the downstream side relative to the conjugate-retaining part, and wherein any one of the first antibody and the second antibody is an anti-oncofetal fibronectin antibody and the other is an antibody binding to oncofetal fibronectin; and
(2) a liquid composition containing a nonionic surfactant represented by the following Formula (1):
R-O-(CH₂CH₂O)ₘH (1)
wherein m is 4 to 30, and R represents an alkyl group.

6. The detection kit of claim 5, wherein the liquid composition of (2) is used as a spreading solution or a specimen extraction solution.

7. The detection kit of claim 5 or 6, wherein a concentration of the surfactant in the liquid composition is in the range of more than 0.2%(W/V) and less than 1.0%(W/V).

8. A liquid composition for detecting oncofetal fibronectin using immunochromatography, comprising a nonionic surfactant represented by the following Formula (1):
R-O-(CH₂CH₂O)ₘH (1)
wherein m is 4 to 30, and R represents an alkyl group.

9. The liquid composition of claim 8, wherein the liquid composition is used as a spreading solution or a specimen extraction solution.

10. The liquid composition of claim 8 or 9, wherein a concentration of the surfactant in the liquid composition is in the range of more than 0.2%(W/V) and less than 1.0%(W/V).

11. A specimen extraction solution for detecting oncofetal fibronectin, comprising a nonionic surfactant represented by the following Formula (1):
R-O-(CH₂CH₂O)ₘH (1)
wherein m is 4 to 30, and R represents an alkyl group.

12. A method of detecting oncofetal fibronectin using immunochromatography, comprising bringing a sample possibly containing oncofetal fibronectin and fibronectins other than oncofetal fibronectin into contact with an anti-oncofetal fibronectin antibody in the presence of a nonionic surfactant represented by the following Formula (1):
R-O-(CH₂CH₂O)ₘH (1)
wherein m is 4 to 30, and R represents an alkyl group.
